(19) Europäisches Patentamt
European Patent Office
Office européen des brevets

(11) Numéro de publication : **0 341 165 B1**

(12) # FASCICULE DE BREVET EUROPEEN

(45) Date de publication du fascicule du brevet : **09.09.92 Bulletin 92/37**

(51) Int. Cl.$^5$ : **C07C 37/60**

(21) Numéro de dépôt : **89420156.5**

(22) Date de dépôt : **27.04.89**

(54) **Procédé de préparation du phénol.**

(30) Priorité : **02.05.88 FR 8805850**

(43) Date de publication de la demande : **08.11.89 Bulletin 89/45**

(45) Mention de la délivrance du brevet : **09.09.92 Bulletin 92/37**

(84) Etats contractants désignés : **BE DE ES FR GB IT NL**

(56) Documents cités :
**EP-A- 0 226 257**
**US-A- 1 547 725**
**THE JOURNAL OF PHYSICAL CHEMISTRY, vol. 88, no. 19, 13 septembre 1984, pages 4195-4197, American Chemical Society; M. IWAMOTOet al.: "Partially zinc ion-exchanged K-A zeolites as molecular sieves distinguishing oxygen from nitrogen"**

(56) Documents cités :
**CHEMISTRY LETTERS, no. 6, 1988, pages 953-956, Chemical Society of Japan; E. SUZUKI: "Hydroxylation of benzene withdinitrogen monoxide over H-ZSM-5 zeolite" (Date de publication: 06-06-1988)**

(73) Titulaire : **RHONE-POULENC CHIMIE
25, quai Paul Doumer
F-92408 Courbevoie Cédex (FR)**

(72) Inventeur : **Gubelmann, Michel
39, Bd des Belges
F-69006 Lyon (FR)**
Inventeur : **Tirel, Philippe-Jean
40 Bd Kennedy
F-69600 Oullins (FR)**

(74) Mandataire : **Dubruc, Philippe et al
RHONE-POULENC CHIMIE Direction de la Propriété Industrielle 25, Quai Paul Doumer
F-92408 Courbevoie Cedex (FR)**

**EP 0 341 165 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation du phénol ; elle concerne plus particulièrement un procédé d'hydroxylation directe du benzène.

Il est connu depuis longtemps (Kirk-Othmer-Thurman C. vol 17, 373-384) divers procédés de synthèses du phenol et tout particulièrement de synthèse à partir de benzène.

Le benzène est mis en contact avec du propylène en présence d'un acide de Lewis tel que le trichlorure d'aluminium ou en milieu hétérogène en présence d'une silice phosphatée.

Le cumène obtenu est oxydé dans une deuxième étape en hydroperoxyde de cumène qui est transformé en milieu sulfurique en phénol. Cette technique est toujours utilisée actuellement mais demande trois étages et passe par des intermédiaires peroxydiques que l'industrie cherche à éviter.

Tous les chimistes ont cherché depuis longtemps à introduire directement sur le noyau benzénique le groupe hydroxyle désiré. Cette chimie a longtemps échoué. La seule publication décrivant l'introduction directe d'un groupe hydroxyle sur un noyau benzénique consiste en un article de IWAMOTO paru dans le Journal of Physical Chemistry, 87, 6, 1983.

Cette réaction d'hydroxylation du benzène est réalisée par le protoxyde d'azote ($N_2O$) en présence d'un catalyseur à base d'un oxyde d'un métal des groupes V ou VI de la classification périodique.

L'oxyde de vanadium est l'oxyde préféré parmi les oxydes des métaux des groupes V et VI de la classification. Il est préférable d'utiliser cet oxyde disposé sur un support à base de silice en quantité pondérale comprise entre 1 et 10 % par rapport au support. Le support est constitué de préférence de silice, l'alumine provoquant majoritairement la formation d'un mélange d'oxydes de carbone.

Le dit procédé était intéressant mais l'utilisation des catalyseurs le rendait peu attrayant pour l'industrie.

L'industrie chimique est donc toujours à la recherche d'un procédé d'hydroxylation directe du noyau benzénique sur un substrat simple et facilement disponible.

La présente invention a permis d'atteindre cet objectif.

Elle a pour objet un procédé de préparation du phénol caractérisé en ce que l'on met en présence du benzène et du protoxyde d'azote sur une zéolithe acidifiée.

La zéolithe est choisie notamment parmi les formes commerciales telles que :

. la zéolithe ZSM-5 de MOBIL-OIL dont la préparation est décrite dans le brevet US 3702886,
. la zéolithe US-Y vendue par TOYO-SODA
. la zéolithe HY vendue par UNION CARBIDE CHEMICALS sous la référence LZY 82.
. la zéolithe H-Mordénite vendue par LA GRANDE PAROISSE

On préfère utiliser la zéolithe commerciale ZSM-5.

La zéolithe a un rapport $SiO_2/Al_2O_3$ supérieur à 90, de préférence compris entre 90 et 500.

La zéolithe commerciale est, de préférence dans le procédé de l'invention, acidifiée par addition d'un acide inorganique choisi parmi l'acide chlorhydrique, l'acide sulfurique, l'acide nitrique, l'acide perchlorique et l'acide phosphorique ou d'un acide organique choisi parmi les acides halosulfoniques, halométhane sulfoniques, halocarboxyliques et de préférence l'acide trifluorométhane-sulfonique.

Les acides organiques ne présentent aucun avantage par rapport aux acides minéraux et présentent surtout l'inconvénient d'être plus onéreux.

Selon un mode de mise en oeuvre préférentiel, la zéolithe est acidifiée par passage d'un volume d'acide, présentant une normalité comprise entre 0,1 N et 2 N, par gramme de zéolithe comprise entre 10 ml/g et 100 ml/g. Ce passage peut être réalisé en une seule étape ou de préférence en plusieurs étapes successives.

Le protoxyde d'azote est utilisé pur ou en mélange avec un gaz inerte ne contenant pas d'oxygène tel que l'azote.

Le benzène est de préférence introduit en mélange avec le protoxyde d'azote selon un rapport molaire, ou volumique du protoxyde d'azote par rapport au benzène compris entre 1 et 10.

Selon un procédé de mise en oeuvre préférentiel, on vaporise le benzène, on le mélange avec le protoxyde d'azote selon les proportions préalablement définies et on le fait circuler sur la zéolithe. La réaction se déroule de préférence à une température comprise entre 300 et 500°C.

Les gaz de réaction contenant le phénol sont condensés.

Les exemples suivants donnés uniquement à titre indicatif et nullement limitatif permettent d'illustrer l'invention.

Dans les exemples, les abréviations suivantes signifient :

$$TT = \text{taux de transformation} = \frac{\text{produit tranformé}}{\text{produit introduit}} \text{ en moles}$$

$$RT = \text{rendement sur produit transformé} = \frac{\text{produit désiré}}{\text{produit transformé}} \text{ en moles}$$

EXEMPLE 1 :

Préparation du catalyseur

On met en contact 10 g de zéolithe commerciale NaZSM5 avec 100 ml d'une solution d'HCl 1N à 60°C pendant 4 heures sous agitation. On laisse refroidir et on lave à l'eau permutée. On filtre le solide et on le sèche à l'étuve à 100°C.

Le lavage ci-dessus décrit est répété 8 fois. Le produit séché obtenu après le 8ème lavage est broyé.

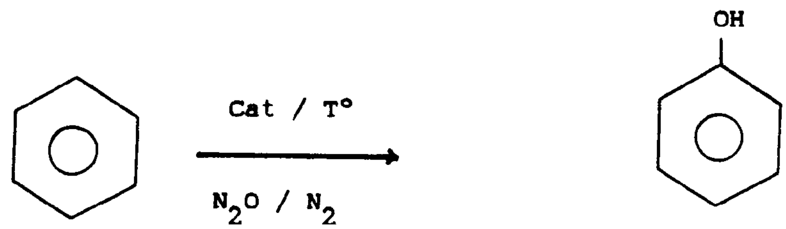


CONDITIONS EXPERIMENTALES :

| | | |
|---|---|---|
| Phase vapeur | : | continue |
| Catalyseur | : | . H Z S M 5 |
| | | . diam pores : 550 pm |
| | | . Rapport $\frac{SiO_2}{Al_2O_3} = 120$ |
| Température | : | Essai 400°C |
| Temps de contact | : | 1 seconde |
| Rapports molaires | : | Benzène / $N_2$ / $N_2O$<br>2 5 8 |

1,05 g (2 ml) de catalyseur HZSM5 ($SiO_2/Al_2O_3$ = 120) en poudre, dispersé dans 4 g de quartz en grains (< 0,8 mm) sont introduits dans un réacteur tubulaire en quartz (longueur = 16 cm, diamètre intérieur = 1,8 cm).

On ajoute ensuite un lit de 10 cm de billes de verre permettant d'homogénéiser le mélange gazeux. Le réacteur ainsi chargé est conditionné 15 heures à 350°C sous azote dans un four tubulaire.

La réaction est effectuée en continu en introduisant 1,4 $cm^3$/h de benzène, 1,44 l/h de protoxyde d'azote et 0,9 l/h d'azote.

Soit en rapport molaire :

Benzène / $N_2$ / $N_2O$
2 5 8

Le taux de transformation du benzène est de 16 %.

La sélectivité en phénol est supérieure à 95 %
(déterminée : 98 %.)

Essai témoin (a) :

On reproduit l'exemple 1 ci-avant à ceci près que le catalyseur HZSM5 présente un rapport $SiO_2/Al_2O_3$ de 50 seulement.

Les résultats obtenus, toutes conditions égales par ailleurs, sont les suivants :

TT = 9,1 %

RT > 95 %

EXEMPLE 2 :

On reproduit l'exemple 1 ci-avant à ceci près que le catalyseur est une zéolithe HNaZS5 présentant un rapport $SiO_2/Al_2O_3$ de 120.

Les résultats obtenus, toutes conditions égales par ailleurs, sont les suivants :

TT = 9,5 %

RT > 95 %

Essai témoin (b) :

On reproduit l'exemple 1 ci-avant à ceci près que le catalyseur est la zéolithe commerciale NaZSM5 présentant un rapport $SiO_2/Al_2O_3$ de 120 non traitée.

Les résultats obtenus, toutes conditions égales par ailleurs, sont les suivants :

TT = 0

## Revendications

1. Procédé de préparation du phénol caractérisé en ce que l'on met en présence du benzène et du protoxyde d'azote sur un zéolithe acidifiée présentant un rapport molaire $SiO_2/Al_2O_3$ supérieur à 90.

2. Procédé selon la revendication 1 caractérisé en ce que la zéolithe acidifiée est choisie parmi les zéolithes HZSM5, HY et H-Mordénite présentant un rapport molaire $SiO_2/Al_2O_3$ compris entre 90 et 500.

3. Procédé selon la revendication 1 caractérisé en ce que la zéolithe est acidifiée par un acide inorganique .

4. Procédé selon la revendication 1 caractérisé en ce que le protoxyde d'azote est utilisé selon un rapport molaire calculé par rapport au benzène compris entre 1 et 10.

5. Procédé selon la revendication 1 caractérisé en ce que la réaction est mise en oeuvre à une température comprise entre 300 et 500°C.

## Patentansprüche

1. Verfahren zur Herstellung von Phenol, dadurch gekennzeichnet, daß man Benzol und Stickoxydul auf einem angesäuerten Zeolithen, der ein Molverhältnis $SiO_2/Al_2O_3$ über 90 aufweist, zusammenbringt.

2. Verfahren nach Anspruch 1, dadurch gekennzeichnet, daß der angesäuerte Zeolith ausgewählt wird aus den Zeolithen HZSM5, HY und H-Mordenit, die ein Molverhältnis $SiO_2/Al_2O_3$ im Bereich von 90 bis 500 aufweisen.

3. Verfahren nach Anspruch 1, dadurch gekennzeichnet, daß der Zeolith mit einer anorganischen Säure angesäuert wird.

4. Verfahren nach Anspruch 1, dadurch gekennzeichnet, daß das Stickoxydul in einem Molverhältnis, bezogen auf Benzol, im Bereich von 1 bis 10 eingesetzt wird.

5. Verfahren nach Anspruch 1, dadurch gekennzeichnet, daß die Reaktion bei einer Temperatur im Bereich von 300 bis 500°C durchgeführt wird.

## Claims

1. Process for the preparation of phenol, characterized in that benzene and nitrous oxide are brought into contact on an acidified zeolite which has a molar ratio $SiO_2/Al_2O_3$, greater than 90.

2. Process according to Claim 1, characterized in that the acidified zeolite is chosen from zeolites HZSM5, HY and H-mordenite which have a molar ratio $SiO_2/Al_2O_3$, of between 90 and 500.

3. Process according to Claim 1, characterized in that the zeolite is acidified with an inorganic acid.

4. Process according to Claim 1, characterized in that nitrous oxide is employed in a molar ratio of between 1 and 10, calculated relative to benzene.

5. Process according to Claim 1, characterized in that the reaction is carried out at a temperature of between 300 and 500°C.